# EUROPEAN PATENT SPECIFICATION

(11) **EP 2 161 287 B1**
(45) Date of publication and mention of the grant of the patent: **04.03.2015**
(21) Application number: 08757443.0
(22) Date of filing: 16.06.2008
(51) Int. Cl.: A61K 38/00, C07K 14/705

(54) **OPTIMIZED TACI-Fc FUSION PROTEINS**
OPTIMIERTE TACI-FC-FUSIONSPROTEINE
PROTEINES DE FUSION TACI-Fc OPTIMISEES

(30) Priority: 15.06.2007 CN 200710111162
(43) Date of publication of application: 10.03.2010
(73) Proprietor: Yantai Rongchang Biotechnologies Co., Ltd., Shandong 264006 (CN)
(72) Inventor: LIU, Zheng, Jiangsu 210018 (CN); FANG, Jianmin, Yantai ETDZ Shandong 264006 (CN)
(74) Representative: Epping - Hermann - Fischer
(86) International application number: PCT/CN2008/001162
(87) International publication number: WO 2008/154814

(56) References cited:
- WO-A1-2006/052493
- WO-A2-01/81417
- WO-A2-02/094852
- WO-A2-2007/019573
- CN-A- 1 612 750
- GROSS J A ET AL: "TACI-IG Neutralizes Molecules Critical for B Cell Development and Autoimmune Disease: Impaired B Cell Maturation in Mice Lacking BLYsS" IMMUNITY, CELL PRESS, US LNKD- DOI:10.1016/S1074-7613(01)00183-2, vol. 15, 1 August 2001 (2001-08-01), pages 289-302, XP003008630 ISSN: 1074-7613
- WU Y. ET AL.: 'Tumor Necrosis Factor (TNF) receptor superfamily member TACI is a high affinity receptor for TNF family members APRIL and BLyS' J. BIOL. CHEM. vol. 275, no. 45, 10 November 2000, pages 35478 - 35485, XP002367757
- HYMOWITZ S.G. ET AL.: 'Structures of APRIL-receptor complexes - like BCMA, TACI employs only a single cysteine-rich domai for high affinity ligand binding' J. BIOL. CHEM. vol. 280, no. 8, 25 February 2005, pages 7218 - 7227, XP002373779
- YAN M.H. ET AL.: 'Identification of a receptor for BLyS demonstrates a crucial role in humoral immunity' NAT. IMMUNOL. vol. 1, no. 1, July 2000, pages 37 - 41, XP002222891

### SEQUENCE LISTING

<110> Yantai RongChang Biotechnologies, Ltd.
<120> Optimized TACI-FC Fusion Protein
<130> P2009,1447 EP N
<140> EP08757443.0-1212
   <141> 2008-06-16
<160> 16
<170> PatentIn version 3.5
<210> 1
   <211> 323
   <212> PRT
   <213> Artificial Sequence
<220>
   <223> fusion protein T1
<400> 1
   Ser Arg Val Asp Gln Glu Glu Arg Phe Pro Gln Gly Leu Trp Thr Gly
   1               5                   10                  15
   Val Ala Met Arg Ser Cys Pro Glu Glu Gln Tyr Trp Asp Pro Leu Leu
               20                  25                  30
   Gly Thr Cys Met Ser Cys Lys Thr Ile Cys Asn His Gln Ser Gln Arg
           35                  40                  45
   Thr Cys Ala Ala Phe Cys Arg Ser Leu Ser Cys Arg Lys Glu Gln Gly
       50                  55                  60
   Lys Phe Tyr Asp His Leu Leu Arg Asp Cys Ile Ser Cys Ala Ser Ile
   65                  70                  75                  80
   Cys Gly Gln His Pro Lys Gln Cys Ala Tyr Phe Cys Glu Asn Lys Leu
                   85                  90                  95
   Asp Lys Pro His Thr Cys Pro Leu Cys Pro Ala Pro Glu Leu Leu Gly
               100                 105                 110
   Gly Pro Ser Val Phe Leu Phe Pro Pro Lys Pro Lys Asp Thr Leu Met
           115                 120                 125
   Ile Ser Arg Thr Pro Glu Val Thr Cys Val Val Val Asp Val Ser His
       130                 135                 140
   Glu Asp Pro Glu Val Lys Phe Asn Trp Tyr Val Asp Gly Val Glu Val
   145                 150                 155                 160
   His Asn Ala Lys Thr Lys Pro Arg Glu Glu Gln Tyr Asn Ser Thr Tyr
                   165                 170                 175
   Arg Val Val Ser Val Leu Thr Val Leu His Gln Asp Trp Leu Asn Gly
   180 185 190
   Lys Glu Tyr Lys Cys Lys Val Ser Asn Lys Ala Leu Pro Ala Pro Ile
   195 200 205
   Glu Lys Thr Ile Ser Lys Ala Lys Gly Gln Pro Arg Glu Pro Gln Val
   210 215 220
   Tyr Thr Leu Pro Pro Ser Arg Asp Glu Leu Thr Lys Asn Gln Val Ser
   225 230 235 240
   Leu Thr Cys Leu Val Lys Gly Phe Tyr Pro Ser Asp Ile Ala Val Glu
   245 250 255
   Trp Glu Ser Asn Gly Gln Pro Glu Asn Asn Tyr Lys Ala Thr Pro Pro
   260 265 270
   Val Leu Asp Ser Asp Gly Ser Phe Phe Leu Tyr Ser Lys Leu Thr Val
   275 280 285
   Asp Lys Ser Arg Trp Gln Gln Gly Asn Val Phe Ser Cys Ser Val Met
   290 295 300
   His Glu Ala Leu His Asn His Tyr Thr Gln Lys Ser Leu Ser Leu Ser
   305 310 315 320
   Pro Gly Lys
<210> 2
   <211> 333
   <212> PRT
   <213> Artificial Sequence
<220>
   <223> fusion protein T2
<400> 2
   Ser Arg Val Asp Gln Glu Glu Arg Phe Pro Gln Gly Leu Trp Thr Gly
   1 5 10 15
   Val Ala Met Arg Ser Cys Pro Glu Glu Gln Tyr Trp Asp Pro Leu Leu
   20 25 30
   Gly Thr Cys Met Ser Cys Lys Thr Ile Cys Asn His Gln Ser Gln Arg
   35 40 45
   Thr Cys Ala Ala Phe Cys Arg Ser Leu Ser Cys Arg Lys Glu Gln Gly
   50 55 60
   Lys Phe Tyr Asp His Leu Leu Arg Asp Cys Ile Ser Cys Ala Ser Ile
   65                  70                  75                  80
   Cys Gly Gln His Pro Lys Gln Cys Ala Tyr Phe Cys Glu Asn Lys Leu
                   85                  90                  95
   Arg Ser Pro Val Asn Leu Pro Pro Glu Leu Asp Lys Pro His Thr Cys
               100                 105                 110
   Pro Leu Cys Pro Ala Pro Glu Leu Leu Gly Gly Pro Ser Val Phe Leu
           115                 120                 125
   Phe Pro Pro Lys Pro Lys Asp Thr Leu Met Ile Ser Arg Thr Pro Glu
       130                 135                 140
   Val Thr Cys Val Val Val Asp Val Ser His Glu Asp Pro Glu Val Lys
   145                 150                 155                 160
   Phe Asn Trp Tyr Val Asp Gly Val Glu Val His Asn Ala Lys Thr Lys
                   165                 170                 175
   Pro Arg Glu Glu Gln Tyr Asn Ser Thr Tyr Arg Val Val Ser Val Leu
               180                 185                 190
   Thr Val Leu His Gln Asp Trp Leu Asn Gly Lys Glu Tyr Lys Cys Lys
           195                 200                 205
   Val Ser Asn Lys Ala Leu Pro Ala Pro Ile Glu Lys Thr Ile Ser Lys
       210                 215                 220
   Ala Lys Gly Gln Pro Arg Glu Pro Gln Val Tyr Thr Leu Pro Pro Ser
   225                 230                 235                 240
   Arg Asp Glu Leu Thr Lys Asn Gln Val Ser Leu Thr Cys Leu Val Lys
                   245                 250                 255
   Gly Phe Tyr Pro Ser Asp Ile Ala Val Glu Trp Glu Ser Asn Gly Gln
               260                 265                 270
   Pro Glu Asn Asn Tyr Lys Ala Thr Pro Pro Val Leu Asp Ser Asp Gly
           275                 280                 285
   Ser Phe Phe Leu Tyr Ser Lys Leu Thr Val Asp Lys Ser Arg Trp Gln
       290                 295                 300
   Gln Gly Asn Val Phe Ser Cys Ser Val Met His Glu Ala Leu His Asn
   305                 310                 315                 320
   His Tyr Thr Gln Lys Ser Leu Ser Leu Ser Pro Gly Lys
                   325                 330
<210> 3
   <211> 342
   <212> PRT
   <213> Artificial Sequence
<220>
   <223> fusion protein T3
<400> 3
   Ser Arg Val Asp Gln Glu Glu Arg Phe Pro Gln Gly Leu Trp Thr Gly
   1               5                   10                  15
   Val Ala Met Arg Ser Cys Pro Glu Glu Gln Tyr Trp Asp Pro Leu Leu
               20                  25                  30
   Gly Thr Cys Met Ser Cys Lys Thr Ile Cys Asn His Gln Ser Gln Arg
           35                  40                  45
   Thr Cys Ala Ala Phe Cys Arg Ser Leu Ser Cys Arg Lys Glu Gln Gly
       50                  55                  60
   Lys Phe Tyr Asp His Leu Leu Arg Asp Cys Ile Ser Cys Ala Ser Ile
   65                  70                  75                  80
   Cys Gly Gln His Pro Lys Gln Cys Ala Tyr Phe Cys Glu Asn Lys Leu
                   85                  90                  95
   Arg Ser Pro Val Asn Leu Pro Pro Glu Leu Gly Gly Gly Gly Gly Gly
               100                 105                 110
   Gly Gly Gly Asp Lys Pro His Thr Cys Pro Leu Cys Pro Ala Pro Glu
           115                 120                 125
   Leu Leu Gly Gly Pro Ser Val Phe Leu Phe Pro Pro Lys Pro Lys Asp
       130                 135                 140
   Thr Leu Met Ile Ser Arg Thr Pro Glu Val Thr Cys Val Val Val Asp
   145                 150                 155                 160
   Val Ser His Glu Asp Pro Glu Val Lys Phe Asn Trp Tyr Val Asp Gly
                   165                 170                 175
   Val Glu Val His Asn Ala Lys Thr Lys Pro Arg Glu Glu Gln Tyr Asn
               180                 185                 190
   Ser Thr Tyr Arg Val Val Ser Val Leu Thr Val Leu His Gln Asp Trp
           195                 200                 205
   Leu Asn Gly Lys Glu Tyr Lys Cys Lys Val Ser Asn Lys Ala Leu Pro
       210                 215                 220
   Ala Pro Ile Glu Lys Thr Ile Ser Lys Ala Lys Gly Gln Pro Arg Glu
   225                 230                 235                 240
   Pro Gln Val Tyr Thr Leu Pro Pro Ser Arg Asp Glu Leu Thr Lys Asn
   245 250 255
   Gln Val Ser Leu Thr Cys Leu Val Lys Gly Phe Tyr Pro Ser Asp Ile
   260 265 270
   Ala Val Glu Trp Glu Ser Asn Gly Gln Pro Glu Asn Asn Tyr Lys Ala
   275 280 285
   Thr Pro Pro Val Leu Asp Ser Asp Gly Ser Phe Phe Leu Tyr Ser Lys
   290 295 300
   Leu Thr Val Asp Lys Ser Arg Trp Gln Gln Gly Asn Val Phe Ser Cys
   305 310 315 320
   Ser Val Met His Glu Ala Leu His Asn His Tyr Thr Gln Lys Ser Leu
   325 330 335
   Ser Leu Ser Pro Gly Lys
   340
<210> 4
   <211> 313
   <212> PRT
   <213> Artificial sequence
<220>
   <223> fusion protein T4
<400> 4
   Ala Met Arg Ser Cys Pro Glu Glu Gln Tyr Trp Asp Pro Leu Leu Gly
   1 5 10 15
   Thr Cys Met Ser Cys Lys Thr Ile Cys Asn His Gln Ser Gln Arg Thr
   20 25 30
   Cys Ala Ala Phe Cys Arg Ser Leu Ser Cys Arg Lys Glu Gln Gly Lys
   35 40 45
   Phe Tyr Asp His Leu Leu Arg Asp Cys Ile Ser Cys Ala Ser Ile Cys
   50 55 60
   Gly Gln His Pro Lys Gln Cys Ala Tyr Phe Cys Glu Asn Lys Leu Arg
   65 70 75 80
   Ser Glu Pro Lys Ser Ser Asp Lys Pro His Thr Cys Pro Leu Cys Pro
   85 90 95
   Ala Pro Glu Leu Leu Gly Gly Pro Ser Val Phe Leu Phe Pro Pro Lys
   100 105 110
   Pro Lys Asp Thr Leu Met Ile Ser Arg Thr Pro Glu Val Thr Cys Val
   115 120 125
   Val Val Asp Val Ser His Glu Asp Pro Glu Val Lys Phe Asn Trp Tyr
   130 135 140
   Val Asp Gly Val Glu Val His Asn Ala Lys Thr Lys Pro Arg Glu Glu
   145 150 155 160
   Gln Tyr Asn Ser Thr Tyr Arg Val Val Ser Val Leu Thr Val Leu His
   165 170 175
   Gln Asp Trp Leu Asn Gly Lys Glu Tyr Lys Cys Lys Val Ser Asn Lys
   180 185 190
   Ala Leu Pro Ala Pro Ile Glu Lys Thr Ile Ser Lys Ala Lys Gly Gln
   195 200 205
   Pro Arg Glu Pro Gln Val Tyr Thr Leu Pro Pro Ser Arg Asp Glu Leu
   210 215 220
   Thr Lys Asn Gln Val Ser Leu Thr Cys Leu Val Lys Gly Phe Tyr Pro
   225 230 235 240
   Ser Asp Ile Ala Val Glu Trp Glu Ser Asn Gly Gln Pro Glu Asn Asn
   245 250 255
   Tyr Lys Ala Thr Pro Pro Val Leu Asp Ser Asp Gly Ser Phe Phe Leu
   260 265 270
   Tyr Ser Lys Leu Thr Val Asp Lys Ser Arg Trp Gln Gln Gly Asn Val
   275 280 285
   Phe Ser Cys Ser Val Met His Glu Ala Leu His Asn His Tyr Thr Gln
   290 295 300
   Lys Ser Leu Ser Leu Ser Pro Gly Lys
   305 310
<210> 5
   <211> 969
   <212> DNA
   <213> Artificial sequence
<220>
   <223> DNA sequence coding for fusion protein T1
<400> 5
   agccgtgtgg accaggagga gcgctttcca cagggcctgt ggacgggggt ggctatgaga 60
   tcctgccccg aagagcagta ctgggatcct ctgctgggta cctgcatgtc ctgcaaaacc 120
   atttgcaacc atcagagcca gcgcacctgt gcagccttct gcaggtcact cagctgccgc 180
   aaggagcaag gcaagttcta tgaccatctc ctgagggact gcatcagctg tgcctccatc 240
   tgtggacagc accctaagca atgtgcatac ttctgtgaga acaagctcga caaacctcac    300
   acatgcccac tgtgcccagc acctgaactc ctggggggac cgtcagtctt cctcttcccc    360
   ccaaaaccca aggacaccct catgatctcc cggacccctg aggtcacatg cgtggtggtg    420
   gacgtgagcc acgaagaccc tgaggtcaag ttcaactggt acgtggacgg cgtggaggtg    480
   cataatgcca agacaaagcc gcgggaggag cagtacaaca gcacgtaccg tgtggtcagc    540
   gtcctcaccg tcctgcacca ggactggctg aatggcaagg agtacaagtg caaggtctcc    600
   aacaaagccc tcccagcccc catcgagaaa accatctcca aagccaaagg gcagccccga    660
   gaaccacagg tgtacaccct gcccccatcc cgggatgagc tgaccaagaa ccaggtcagc    720
   ctgacctgcc tagtcaaagg cttctatccc agcgacatcg ccgtggagtg ggagagcaat    780
   gggcagccgg agaacaacta caaggccacg cctcccgtgc tggactccga cggctccttc    840
   ttcctctaca gcaagctcac cgtggacaag agcaggtggc agcaggggaa cgtcttctca    900
   tgctccgtga tgcatgaggc tctgcacaac cactacacgc agaagagcct ctccctgtct    960
   ccgggtaaa                                                            969
<210> 6
   <211> 999
   <212> DNA
   <213> Artificial Sequence
<220>
   <223> DNA sequence coding for fusion protein T2
<400> 6
   agccgtgtgg accaggagga gcgctttcca cagggcctgt ggacgggggt ggctatgaga     60
   tcctgccccg aagagcagta ctgggatcct ctgctgggta cctgcatgtc ctgcaaaacc    120
   atttgcaacc atcagagcca gcgcacctgt gcagccttct gcaggtcact cagctgccgc    180
   aaggagcaag gcaagttcta tgaccatctc ctgagggact gcatcagctg tgcctccatc    240
   tgtggacagc accctaagca atgtgcatac ttctgtgaga acaagctcag gagcccagtg    300
   aaccttccac cagagctcga caaacctcac acatgcccac tgtgcccagc acctgaactc    360
   ctggggggac cgtcagtctt cctcttcccc ccaaaaccca aggacaccct catgatctcc    420
   cggacccctg aggtcacatg cgtggtggtg gacgtgagcc acgaagaccc tgaggtcaag    480
   ttcaactggt acgtggacgg cgtggaggtg cataatgcca agacaaagcc gcgggaggag    540
   cagtacaaca gcacgtaccg tgtggtcagc gtcctcaccg tcctgcacca ggactggctg    600
   aatggcaagg agtacaagtg caaggtctcc aacaaagccc tcccagcccc catcgagaaa    660
   accatctcca aagccaaagg gcagccccga gaaccacagg tgtacaccct gcccccatcc    720
   cgggatgagc tgaccaagaa ccaggtcagc ctgacctgcc tagtcaaagg cttctatccc    780
   agcgacatcg ccgtggagtg ggagagcaat gggcagccgg agaacaacta caaggccacg    840
   cctcccgtgc tggactccga cggctccttc ttcctctaca gcaagctcac cgtggacaag    900
   agcaggtggc agcaggggaa cgtcttctca tgctccgtga tgcatgaggc tctgcacaac    960
   cactacacgc agaagagcct ctccctgtct ccgggtaaa 999
<210> 7
   <211> 1026
   <212> DNA
   <213> Artificial Sequence
<220>
   <223> DNA sequence coding for fusion protein T3
<400> 7
   agccgtgtgg accaggagga gcgctttcca cagggcctgt ggacgggggt ggctatgaga      60
   tcctgccccg aagagcagta ctgggatcct ctgctgggta cctgcatgtc ctgcaaaacc     120
   atttgcaacc atcagagcca gcgcacctgt gcagccttct gcaggtcact cagctgccgc     180
   aaggagcaag gcaagttcta tgaccatctc ctgagggact gcatcagctg tgcctccatc     240
   tgtggacagc accctaagca atgtgcatac ttctgtgaga acaagctcag gagcccagtg     300
   aaccttccac cagagctcgg tggaggtgga ggtggaggtg gaggtgacaa acctcacaca     360
   tgcccactgt gcccagcacc tgaactcctg gggggaccgt cagtcttcct cttcccccca     420
   aaacccaagg acaccctcat gatctcccgg acccctgagg tcacatgcgt ggtggtggac     480
   gtgagccacg aagaccctga ggtcaagttc aactggtacg tggacggcgt ggaggtgcat     540
   aatgccaaga caaagccgcg ggaggagcag tacaacagca cgtaccgtgt ggtcagcgtc     600
   ctcaccgtcc tgcaccagga ctggctgaat ggcaaggagt acaagtgcaa ggtctccaac     660
   aaagccctcc cagcccccat cgagaaaacc atctccaaag ccaaagggca gccccgagaa     720
   ccacaggtgt acaccctgcc cccatcccgg gatgagctga ccaagaacca ggtcagcctg     780
   acctgcctag tcaaaggctt ctatcccagc gacatcgccg tggagtggga gagcaatggg     840
   cagccggaga acaactacaa ggccacgcct cccgtgctgg actccgacgg ctccttcttc     900
   ctctacagca agctcaccgt ggacaagagc aggtggcagc aggggaacgt cttctcatgc     960
   tccgtgatgc atgaggctct gcacaaccac tacacgcaga agagcctctc cctgtctccg    1020
   ggtaaa                                                               1026
<210> 8
   <211> 939
   <212> DNA
   <213> Artificial Sequence
<220>
   <223> DNA sequence coding for fusion protein T4
<400> 8
   gctatgagat cctgccccga agagcagtac tgggatcctc tgctgggtac ctgcatgtcc      60
   tgcaaaacca tttgcaacca tcagagccag cgcacctgtg cagccttctg caggtcactc     120
   agctgccgca aggagcaagg caagttctat gaccatctcc tgagggactg catcagctgt     180
   gcctccatct gtggacagca ccctaagcaa tgtgcatact tctgtgagaa caagctcagg     240
   agcgagccca aatcttcaga caaacctcac acatgcccac tgtgcccagc acctgaactc     300
   ctggggggac cgtcagtctt cctcttcccc ccaaaaccca aggacaccct catgatctcc     360
   cggacccctg aggtcacatg cgtggtggtg gacgtgagcc acgaagaccc tgaggtcaag     420
   ttcaactggt acgtggacgg cgtggaggtg cataatgcca agacaaagcc gcgggaggag     480
   cagtacaaca gcacgtaccg tgtggtcagc gtcctcaccg tcctgcacca ggactggctg     540
   aatggcaagg agtacaagtg caaggtctcc aacaaagccc tcccagcccc catcgagaaa     600
   accatctcca aagccaaagg gcagccccga gaaccacagg tgtacaccct gcccccatcc     660
   cgggatgagc tgaccaagaa ccaggtcagc ctgacctgcc tagtcaaagg cttctatccc     720
   agcgacatcg ccgtggagtg ggagagcaat gggcagccgg agaacaacta caaggccacg     780
   cctcccgtgc tggactccga cggctccttc ttcctctaca gcaagctcac cgtggacaag     840
   agcaggtggc agcaggggaa cgtcttctca tgctccgtga tgcatgaggc tctgcacaac     900
   cactacacgc agaagagcct ctccctgtct ccgggtaaa                            939
<210> 9
   <211> 21
   <212> DNA
   <213> Artificial sequence
<220>
   <223> forward primer for fusion protein T1
<400> 9
   agccgtgtgg accaggagga g 21
<210> 10
   <211> 23
   <212> DNA .
   <213> Artificial Sequence
<220>
   <223> reverse primer for fusion protein T1
<400> 10
   gagcttgttc tcacagaagt atg 23
<210> 11
   <211> 21
   <212> DNA
   <213> Artificial sequence
<220>
   <223> forward primer for fusion protein T2
<400> 11
   agccgtgtgg accaggagga g 21
<210> 12
   <211> 23
   <212> DNA
   <213> Artificial Sequence
<220>
   <223> reverse primer for fusion protein T2
<400> 12
   gagctctggt ggaaggttca ctg 23
<210> 13
   <211> 21
   <212> DNA
   <213> Artificial Sequence
<220>
   <223> forward primer for fusion protein T3
<400> 13
   agccgtgtgg accaggagga g 21
<210> 14
   <211> 57
   <212> DNA
   <213> Artificial Sequence
<220>
   <223> reverse primer for fusion protein T3
<400> 14
   acctccacct ccacctccac ctccaccgag ctctggtgga aggttcactg ggctcct 57
<210> 15
   <211> 22
   <212> DNA
   <213> Artificial Sequence
<220>
   <223> forward primer for fusion protein T4
<400> 15
   gctatgagat cctgccccga ag 22
<210> 16
   <211> 20
   <212> DNA
   <213> Artificial Sequence
<220>
   <223> reverse primer for fusion protein T4
<400> 16
   tgaagatttg ggctcgctcc 20

## Description

### FIELD OF THE INVENTION

The invention relates to the preparation of an optimized fusion protein, particularly a TACI-Fc fusion protein, by using genetic recombination technology and its application for the treatment of immunological diseases.

### BACKGROUND OF THE INVENTION

The process of lymphocyte development and differentiation is tightly controlled by various cytokines. B lymphocyte stimulator (BLyS, or so-called BAFF, TNSF13B, THANK, TALL-1, zTNF4) and a proliferation-inducing ligand (APRIL) are cytokines that play important roles in regulating immune response in vivo (Schneider, 2005, Current Opinion in Immunology, 17:282-289; Seyler et al., 2005, The Journal of Clinical Investigation, 115:3083-3092). They stimulate B lymphocyte development and proliferation to increase the expression of various immunoglobulins in the blood. BLyS and APRIL also have essential regulating effects on the maturity of T lymphocytes and thus on cellular immunity (Wang et al., 2001, Nature Immunology, 2:632-637).

BLyS and APRIL regulate immune response of lymphocytes through the receptors on cell surfaces of lymphocytes. They bind to cell membrane receptors TACI (Transmembrane activator and CAML-interactor) and BCMA (B cell maturation antigen). In addition, BLyS can also bind to another receptor, BAFF-R. B lymphocyte expresses TACI, BCMA and BAFF-R. Mature T lymphocyte expresses TACT. BLyS and APRIL regulate the activation, proliferation and development of lymphocytes through signal tranaductions mediated by these receptors, and thus enhance immune responses. In addition, in lymphocyte-derived tumors, BLyS and APRIL also promote tumor cell proliferation and suppress apoptosis, thereby accelerate tumor progression.

Excess expression of BLyS and APRIL is one of the pathogenesis factors for many autoimmune diseases, which include but not limited to systemic lupus erythematosus (SLE), rheumatoid arthritis (RA), Sjogren syndrome, etc. It is shown in clinical investigations that the concentration of BLyS is often positively correlated to the severity of autoimmune diseases. Therefore, the inhibition of BLyS and APRIL has become a potentially effective means for the treatment of related autoimmune diseases. Meanwhile, since BLyS and APRIL can accelerate the progression of B lymphocyte tumor, the inhibition of BLyS and APRIL can also be used to treat B lymphocyte tumors, such as chronic lymphocyte leukemia, multiple myeloma, B lymphocyte lymphoma, etc.

Since TACI has high affinity to each of BLyS and APRIL, it is possible to use soluble TACI (extracellular portion of TACI) to prevent the interactions of BLyS or APRIL with their membrane receptors (TACI, BCMA, BAFF-R), thereby blocking biological activity of BLyS or APRIL and treating autoimmune diseases or tumors.

It is shown in studies that the fusion protein of TACI extracellular portion and IgG Fc fragment (TACI-Fc) can effectively inhibit disease progression caused by BLyS (Gross et al., 2001, Immunity, 15:289-302). However, it is also revealed in studies that the TACI-Fc fusion protein constructed by using extracellular portion of native TACI is easy to be degraded when expressed in cells, and the products obtained thereby are often heterogeneous. Therefore, the TACI-Fc fusion protein of native TACI with Fc fragment is not suitable for industrial production. This invention is aimed at improving TACI-Fc fusion protein by genetic engineering technology so as to develop a pharmaceutical composition applicable for industrial production.

### SUMMARY OF THE INVENTION

The invention relates to methods for the construction and production of a protein drug that blocks BLyS and APRIL, preparation method of the same and use of the same for the treatment of diseases.

The invention provides several fusion proteins which are obtained by the fusion of TACI fragment with immunoglobulin Fc fragment and can block the proliferation of B lymphocyte, the structure of the fusion protein is characterized in that:
the TACI portion of the fusion protein consists of a partial sequence of amino-terminal region of extracellular region, the whole sequence of cysteine-rich region and a partial sequence of stalk region from TACI; while the immunoglobulin Fc in the fusion protein comprises hinge region, CH2 region and CH3 region; the TACI sequence and the Fc sequence are fused directly or via a linker sequence
   (1) wherein the PC cleavage sites at the position of 9^{th}, 12^{th}, 120^{th} and 135^{th} amino acids have been removed through truncation in the TACI portion of the fusion protein, and
   (2) wherein the fusion protein has the function of blocking BLyS and APRIL;
      wherein the TACI fragment may be selected from the group consisting of the positions from 13 to 108 or from 13 to 118 in the amino acid sequence of TACI, and the said linker sequence may be 9Gly, and
      the immunoglobulin Fc fragment may be derived from the Fc of human or animal immunoglobulin, and may be full-length Fc or its partial sequence; and additionally Fc may be derived from IgG, IgM, IgD, and IgA, each of which includes all isotypes respectively, such as IgG1, IgG2, IgG3, and IgG4, and preferably IgG1; and
      wherein said TACI fragment may be selected from the group consisting of the TACI sequences from human and animals.

In particular, a preferable fusion protein of the invention may be:
a. fusion protein T1: which is produced by fusing the 13th -108th amino acids of TACI and immunoglobulin IgG1 Fc (SEQ ID NO: 1),
b. fusion protein T2: which is produced by fusing the 13th -118th amino acids of TACI and immunoglobulin IgG1 Fc (SEQ ID NO: 2), and
c. fusion protein T3: which is produced by fusing the 13th -118th amino acids of TACI, a linker sequence of 9Gly and immunoglobulin IgG1 Fc (SEQ ID NO: 3); and
wherein the TACI is selected from the group consisting of the sequences of extracellular regions for the TACI (transmembrane activator and CAML interactor) from human and animals.

The invention also relates to an DNA sequence which encodes the protein of the invention.

The said DNA sequence is illustrated in SEQ ID NO: 5 to 7.

The TACI-Fc fusion protein with native TACI sequence is not applicable for industrial production due to easy protein degradation. PCT/US2002/015910 describes that the fusion of the TACI fragment, in which the whole amino terminal region and a partial sequence of the stalk region are deleted, with Fc fragment can prevent protein degradation of the fusion protein. However, it was reported that the removal of the amino terminal region significantly reduces biological activity of TACI. (Wu et al., 2000, The Journal of Biological Chemistry, 275:35478-35485).

The key of the invention lies in that a computer software system of Proprotein convertase (PC) artificial neural networks is utilized to analyze the amino acid sequence of TACI extracellular region. The analysis result showed that the sequence of TACI extracellular region includes two PC cleavage sites (the 9th and 135th amino acid), and two additional sites (the 12th and 120th amino acid) have a score close to the threshold value for PC cleavage site. It is presumed that these PC cleavage sites may be essentially responsible for the degradation of TACI in the process of the expression. Based on this discovery, we removed these PC cleavage sites in the optimization of TACI molecule and fused the TACI fragments without PC cleavage sites to Fc. Our experiment proved that new fusion protein effectively prevents the degradation of TACI during the expression. A truncated TACI sequence as well as other methods may be used to prevent these PC cleavage sites. For example, DNA site-directed mutagenesis may be used to remove these PC cleavage sites. Thus various optimization methods can be chosen to achieve the same purpose. The optimized TACI-Fc fusion protein of the invention retains most residues in the amino terminal region and a long amino acid sequence in the stalk region of the TACI molecule. The optimized TACI-Fc fusion protein overcomes the issue of protein degradation. Compared with the TACI-Fc fusion in which the entire N-terminal region has been removed, the optimized TACI-Fc fusion proteins of the invention showed higher biological activity and protein expression level.

The construction of fusion proteins in the invention is based on conventional molecular cloning technology, and specific experimental protocols may be referred to various laboratory manuals, such as the 2nd and 3rd editions of Molecular Cloning: A Laboratory Manual.

PCR synthesis method was used to clone a DNA encoding the above fusion protein into a vector. The vector for expressing the fusion protein may be a plasmid commonly used in molecular biology. A signal peptide sequence is included at the front of the amino terminus of the fusion protein so as to insure protein secretion from cells. The vector sequence includes a promoter for driving gene expression, start and stop signals for protein translation, as well as polyadenylation (polyA) sequence. The vector may include an antibiotics resistance gene to facilitate plasmid replication in bacteria. In addition, the vector may also include an eukaryotic cell selection gene for the selection of stable transfected ceil strains.

After the construction of plasmid, DNA sequence of the fusion protein is confirmed by sequencing. Then corresponding protein can be expressed by the transfection of the plasmid DNA into cells. Many expression systems are available for expressing such fusion proteins, which include but not limited to mammalian cells, bacteria, yeast, insect cells, etc.

Proteins expressed in mammalian cells are glycosylated in post-translational modification process. The amino acid sequences of the TACI-Fc fusion proteins of this invention contain glycosylated amino acids; so mammalian cells are most preferable to express these proteins. There are various mammalian cells suitable for large-scale protein expression, such as CHO cells, SP20 cells, NS0 cells, COS cells, BHK cells, and PerC6 cells. Many other cells can also be used for the expression and production of these proteins, and thus are contemplated in the invention. The plasmid encoding polypeptides can enter cells by transfection. Many methods for cell transfection, and include (but not limited to) electroporation, and liposome- or calcium-mediated transfection, etc.

In addition to mammalian cells, other expression systems may also be used for the expression of these fusion proteins, such as bacterium, yeast and insect cells, which are also contemplated in the invention. The protein yield in these expression systems may be higher than that in mammalian cells, but the protein produced in these expression systems generally lacks glycosylation, or has a form of sugar conjugate which is different from that in mammalian cells.

After protein expression, enzyme-linked immunosorbent assay (ELISA) or other methods may be used to determine the concentrations of the fusion proteins in cell culture supernatants. Because these fusion proteins contain the Fc fragment of immunoglobulin, protein A or G affinity chromatography may be used as an initial purification step to isolate these fusion proteins.

The fundamental function of the TACI-Fc fusion protein of the invention is to block BLyS and APRIL, while BLyS and APRIL are key factors for stimulating B-lymphocyte development and T-lymphocyte maturation. Accordingly, the TACI-Fc fusion protein can be used in diseases related with abnormal immunological function and diseases caused by abnormal proliferation of B-lymphocyte. These diseases include (but not limited to) rheumatoid arthritis, systemic lupus erythematosus, and B lymphocyte tumor. The TACI-Fc fusion protein may be injected into human body as purified recombinant protein. Alternatively, the DNA sequence encoding the fusion protein may be inserted into a proper vector to express the fusion protein in patient's body by means of gene therapy or cell therapy. Therefore, there are many approaches to use the fusion protein of the invention, which include the use of not only the protein by itself, but also the DNA which encodes the fusion protein.

The invention also relates to a pharmaceutical composition of the fusion protein, which may comprises a pharmaceutically acceptable carrier. The pharmaceutical composition may be any form of pharmaceutical formulations, preferably formulations for injection, including liquid form and lyophilized form. The pharmaceutical composition can be prepared according to conventional pharmaceutical methodology, which comprises mixing the active ingredient, i.e. the fusion protein of this invention, with a pharmaceutically acceptable carrier to prepare a desired dosage form based on pharmaceutical technology.

### BRIEF DESCRIPTION OF THE DRAWINGS

Figure 1 shows the analysis of TACI amino acid sequence by proprotein convertase (PC) artificial neural networks. The 9th and 135th amino acid residues in TACI extracellular region are indicated as PC cleavage sites. The 12th and 120th amino acid residues also have a close value to the threshold for PC cleavage site.
Figure 2 shows the structures of native full-length TACI protein and four TACI-Fc fusion proteins.
Figure 3 shows the comparison of protein expression from CHO cells for the four TACI-Fc fusion proteins.
Figure 4 shows the assay for Binding of four TACI-Fc fusion proteins to BLyS. The results show that all the fusion protein can bind to BLyS, in which T3 has the highest affinity to BLyS.
Figure 5 shows SDS-PAGE analysis of purified T3 fusion protein. T3 fusion protein expressed in a high performance CHO expression system appeared as a single protein band in the gel, and has an apparent molecular weight of about 42 kD, which is consistent with the molecular weight expectation of glycosylated T3 fusion protein.
Figure 6 shows significant effect against autoimmune diseases of T3 fusion protein in collagen-induced mouse arthritis model. The progression t of mouse arthritis was measured by arthritis clinical scores. The results show that the mice treated with the TACI-Fc fusion protein T3 has significantly lower clinical scores than that of control group (P<0.01).

### DETAILED DESCRIPTION OF THE INVENTION

The invention provides the following examples for detailedly illustrating the design, construction and evaluation of the TACI-Fc fusion proteins. However, the content and use of the invention are not limited to the scope of the examples.

### Example 1: Sequence analysis of TACI

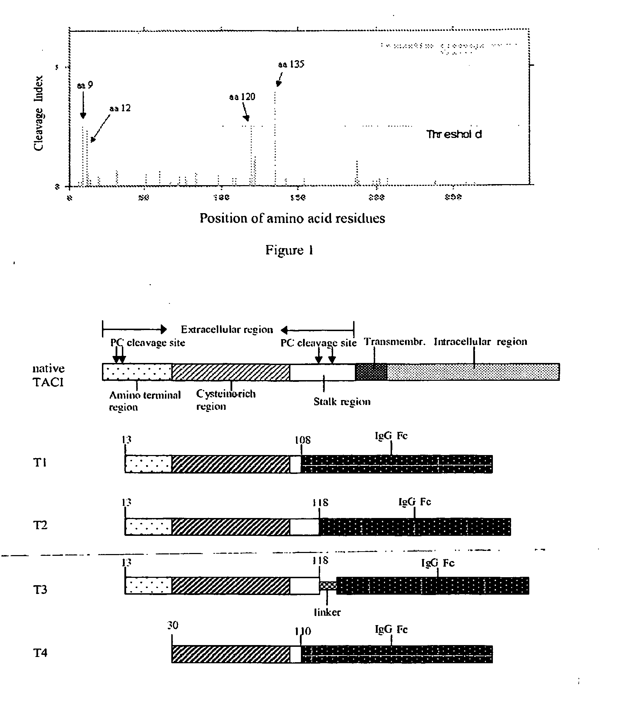

Proprotein convertase (PC) can specifically cleave polypeptides in cells. For many proteins, PC cleavage is an important step during protein post-translational modification. The PC family consists of a number of enzymes, which recognize and cleave specific amino acid sequences. PC artificial neural networks is a computer software system that utilizes a database for known PC cleavage sites to predict PC cleavage sites existing in a protein sequence. The system can be utilized to analyze any potential PC cleavage sites in a protein. The invention analyzed TACI amino acid sequence by using the PC artificial neural network, and it is found that two PC cleavage sites (the 9th and 135th amino acid) exist in the TACI extracellular region. In addition, two additional sites (the 12th and 120th amino acid) have the scores close to the threshold for PC cleavage site (Figure 1). Therefore, it is possible to design an optimized TACI-Fc fusion protein by removing these PC cleavage sites and fusing TACI sequence without potential PC cleavage sites to Fc, so as to prevent protein cleavage. Based on these findings, we have constructed several TACI-Fc fusion proteins, wherein the TACI sequences were derived from the native TACI sequence between the 12th and 120th amino acid (Figure 2). The common characteristics shared by these novel TACI-Fc fusion proteins are that: 1) the major sequence of amino terminal region of TACI is comprised, 2) the entire sequence of cysteine-rich region is comprised, and 3) the partial sequence of the stalk region is comprised.

### Example 2. Construction of fusion proteins and plasmids encoding the same

Total RNA was isolated and purified from human peripheral blood mononuclear cells by using Qiagen total RNA purification kit. cDNA was synthesized from the total RNA with reverse transcriptase. Then the desired TACI fragments were obtained by polymeras chain reaction (PCR) amplification with proper primers. The immunoglobulin Fc fragment was obtained by PCR amplification from a cloned IgG1 Fc plasmid. Finally, the TACI and Fc sequences were fused by PCR, and consequently the DNA sequences of TACI-Fc fusion proteins were successfully constructed.

The PCR reactions comprise: 30 cycles of denaturing at 95°C for 30 seconds; annealing at 56°C for 45 seconds; and extending at 72°C for 2 min. The PCR products of TACI and Fc were cloned respectively into pCR2.1 plasmid by using TA cloning kit. The plasmids were transformed into E. coli. White E. coli colonies were selected and grown in LB medium overnight. Then the plasmids were isolated by using Qiagen plasmid kit. The TACI and Fc sequences were confirmed through restriction enzyme digestions and DNA sequencing. Finally, the TACI and IgG Fc cDNA were fused by using an overlapping PCR method. The TACI-Fc fusion fragments were inserted into a mammalian expression plasmid, the recombinant plasmids were transformed into E. coli, positive colonies were selected, and the target sequences were confirmed through restriction enzyme digestion and DNA sequencing analyses. Finally, the resulted plasmids were used to transfect CHO cells to express the fusion proteins.

| | | |
|---|---|---|
| T1 protein | amino acids 13-108 of TACI + Fc | SEQ ID NOs: 1 and 5 |
| T2 protein | amino acids 13-118 of TACI + Fc | SEQ ID NOs: 2 and 6 |
| T3 protein | amino acids 13-118 of TACI + linker sequence 9Gly + Fc | SEQ ID NOs: 3 and 7 |

In addition, this study also included a fusion protein T4 for comparison. The difference of T4 protein from other three fusion proteins is the absence of TACI N-terminal region in T4. In fact, T4 is a fusion protein between TACI amino acid residues 30-110 and Fc sequence (See SEQ ID NOs: 4 and 8).

Primer sequences for T1 fusion protein:
Forward primer: AGCCGTGTGGACCAGGAGGAG
Reverse primer: GAGCTTGTTCTCACAGAAGTATG

Primer sequences for T2 fusion protein:
Forward primer: AGCCGTGTGGACCAGGAGGAG
Reverse primer: GAGCTCTGGTGGAAGGTTCACTG

Primer sequences for T3 fusion protein:
Forward primer: AGCCGTGTGGACCAGGAGGAG
Reverse primer:

Primer sequences T4 fusion protein:
Forward primer: GCTATGAGATCCTGCCCCGAAG
Reverse primer: TGAAGATTTGGGCTCGCTCC

### Example 3: Expression of TACI-Fc fusion proteins in cells.

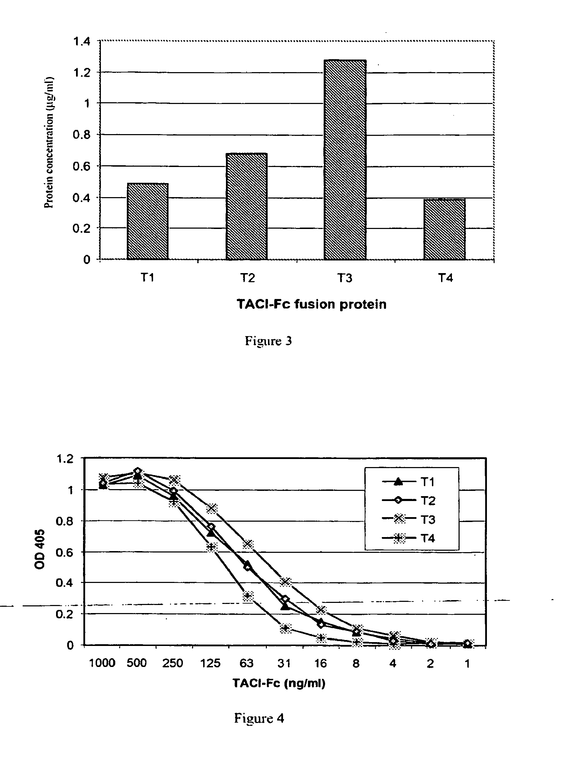

The invention illustrated the expression of TACI-Fc fusion proteins in mammalian cells for example. Four fusion proteins T1, T2, T3, and T4 were cloned into pcDNA3.1 plasmid (Invitrogen). An Flt-1 signal peptide sequence was added at 5' end of each fusion protein. Four TACI-Fc plasmids were purified using high purity plasmid DNA purification kit (QIAGEN). Then the plasmid DNA was introduced into CHO cells respectively with FUGEN6 transfection kit (ROCHE). Transient transfection method was used to compare expression levels of the four TACI-Fc fusion proteins. CHO cells were cultured in cell culture plates in complete DMEM medium with 10% fetal calf serum. When the cells reached to a certain density, the complex of plasmid DNA with FUGEN 6 reagent (Roche) was added to the culture. After culturing for three days, the supernatants were harvested, which contains expressed fusion proteins. The concentrations of fusion proteins were determined with ELISA method. The results of protein concentrations were shown in Figure 3: T3 has the highest expression *inter alia*, followed by T2. T4 has the lowest expression level. Thus, in view of the expression of protein, the fusion protein T3 is the best molecular composition.

### Example 4: Evaluation of binding activities of fusion protein to BLyS.

After the expression of each fusion protein, we determined their binding activity to BLyS in a BLyS binding assay. In this assay, recombinant BLyS protein (R&D Systems) was firstly coated onto 96-well ELISA plates. Nonspecific protein binding sites were blocked with 2% BSA blocking solution. The TACI fusion proteins in various concentrations were then added into respective wells. The plates were incubated for two hours at 37°C and then washed before the addition of rabbit anti-human Ig antibody-HRP conjugate. Finally, peroxidase substrate was used for color development. An ELISA plate reader was used to measure OD value for each well of the 96-well plate, in which an increased OD value indicates the binding of the fusion protein to BLyS. We found that T3 has the best binding to BlyS, T1 and T2 have moderate bindings, and T4 has the lowest binding (Figure 4). Thus, in view of both protein expression and binding activity to BLyS, the fusion protein T3 is the best molecular composition.

### Example 5: Establishment of high expression cell lines.

After determining that the fusion protein T3 is the best molecular composition, we established a stable and high expression strain of CHO cells by using stable transfection and gene amplification. The gene coding for the fusion protein T3 was cloned into a plasmid containing DHFR gene. High purity plasmid DNA was purified by using QIAGEN plasmid purification kit, and CHO cells were transfected with the plasmid using electroporation. The transfected cells were cultured in selection medium to obtain resistant clones. Methotrexate (MTX) was added to amplify T3 gene and obtain high expression cell clones. Finally the CHO cells of the selected clone were expanded in culture, and the fusion protein was produced in a large scale in the bioreactor. The concentration of the fusion protein was determined by ELISA. Using this approach, we have successfully produced the fusion protein T3 in a large scale. The yield of the fusion protein may be higher than 20 pg per cell per day. The fusion protein T3 may be purified by using protein A or G affinity chromatography.

### Example 7: Electrophoretic identification of TACI-Fc fusion protein

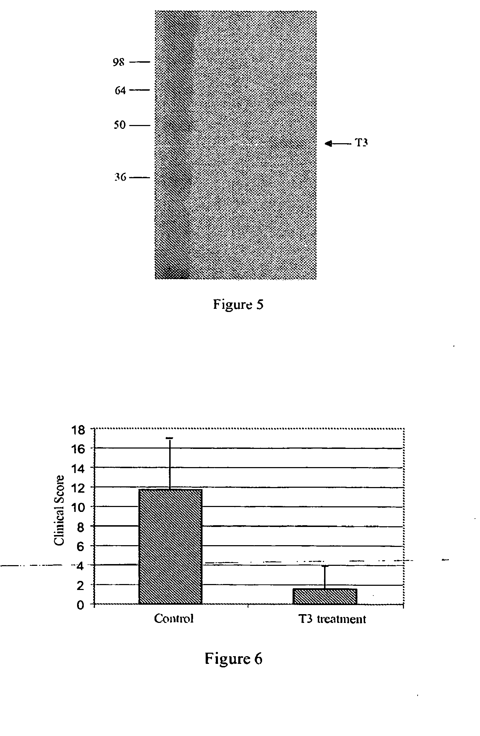

The invention demonstrated experimentally that no degradation of fusion protein occurs during the expression of the optimized fusion protein. The strain of CHO cells for high expression of fusion protein T3 was cultured in cell culture flask. After cells reached high density, the cell culture supernatant was harvested and the fusion protein was purified by using protein A affinity chromatography. The purified fusion protein was mixed with SDS-PAGE loading buffer and boiled for 3 minutes. The fusion protein was separated in polyacrylamide gel and stained with Coomassie brilliant blue. Protein bands were visualized after the gel was destained with methanol. It was shown that T3 protein appeared as a single band in the gel (Figure 5), with an apparent molecular weight of about 42 kD, which is consistent with the molecular weight expectation of glycosylated T3 fusion protein. It is important that no protein band at the molecular weights less than 42 kD were observed. This result demonstrated that the optimized TACI-Fc fusion protein of this invention can effectively prevent protein cleavage and degradation in the process of expression, and thus can be used for large-scale industrial production.

### Example 8: Therapeutic effects of the optimized TACI-Fc fusion protein on autoimmune disease

It was demonstrated in collagen-induced mouse arthritis model that the optimized TACI-Fc fusion protein has anti-autoimmune biological activity. DBA/1 mice at the age of 8 weeks were divided into two groups with 10 animals in each group. Mice were each injected intracutaneously with 200 µg of bovine collagen type II mixed with complete Freund's adjuvant. After 21 days, mice were each injected intracutaneously with bovine collagen protein type II mixed with incomplete Freund's adjuvant. From day 24, mice in test group were injected subcutaneously each with 100 µg of T3 fusion protein for three times a week. Mice in control group were injected with normal human IgG at the same dosage. Progression of arthritis was measured using clinical scores of arthritis. It was shown that the severity of arthritis in the T3-treated mice is significantly less than that in the control mice and there was a significant difference (P<0.01) in clinical scores between the two groups (Figure 6).

In summary, the optimized TACI-Fc fusion proteins of the invention prevent protein degradation in the process of expression, have stronger biological activity, and show high protein expression levels, thus they can be used in large-scale industrial production.

## Claims

1. A fusion protein comprised of truncated TACI and immunoglobulin Fc, **characterized in that** the TACI portion of the fusion protein consists of a sequence of the amino terminal region of extracellular region starting from the 13th amino acid residue, the whole sequence of cysteine-rich region, and a partial sequence of stalk region from TACI; the immunoglobulin Fc portion of the fusion protein comprises hinge region, CH2 region and CH3 region; and the TACI portion and the Fc portion are fused either directly or via a linker sequence,
(1) wherein the PC cleavage sites at the position of 9^{th}, 12^{th}, 120^{th} and 135^{th} amino acids have been removed through truncation in the TACI portion of the fusion protein, and
(2) wherein the fusion protein has the function of blocking BLyS and APRIL.

2. The fusion protein according to Claim 1, **characterized in that** the TACI portion is selected from the group consisting of the 13th to 108th amino acids and the 13th to 118th amino acids of TACI; the linker sequence is 9Gly; and the immunoglobulin Fc portion is derived from the Fc of human or animal immunoglobulins IgG, IgM, IgD, and IgA including all isotypes of each.

3. The fusion protein according to Claim 1, **characterized in that** the immunoglobulin Fc portion is derived from IgG1.

4. The fusion protein according to Claim 1, **characterized in that** the amino acid sequence of the fusion protein is selected from the sequences set forth in SEQ ID NOs: 1 to 3.

5. A DNA sequence encoding the fusion protein according to Claim 1.

6. The DNA sequence according to Claim 5, **characterized in that** it is the sequence set forth in SEQ ID NOs: 5 to 7.

7. A vector comprising the DNA sequence according to Claim 5.

8. A cell comprising the vector according to Claim 7, which are used for the expression of the corresponding fusion protein according to Claim 1, and are selected from eukaryotic cell or prokaryotic cell.

9. Use of the fusion protein according to Claim 1 in the manufacture of a medicament for blocking BLyS or APRIL.

10. A pharmaceutical composition comprising the fusion protein according to Claim 1.

## Patentansprüche

1. Fusionsprotein, umfassend verkürztes TACI und Immunglobulin-Fc, **dadurch gekennzeichnet, dass** der TACI-Anteil des Fusionsproteins aus einer Sequenz des aminoterminalen Bereichs des extrazellulären Bereichs, beginnend vom 13. Aminosäurerest, der ganzen Sequenz des cysteinreichen Bereichs und einer Teilsequenz des Stielbereichs (Stalk) von TACI besteht; der Immunglobulin-Fc-Anteil des Fusionsproteins Scharnierbereich (Hinge), CH2-Bereich und CH3-Bereich umfasst; und der TACI-Anteil und der Fc-Anteil entweder direkt oder über eine Linker-Sequenz fusioniert sind,
(1) wobei die PC-Spaltstellen an den Positionen der 9., 12., 120. und 135. Aminosäure über die Verkürzung im TACI-Anteil des Fusionsproteins entfernt wurden und
(2) wobei das Fusionsprotein die Funktion der Blockierung von BLyS und APRIL aufweist.

2. Fusionsprotein nach Anspruch 1, **dadurch gekennzeichnet, dass** der TACI-Anteil aus der aus der 13. bis 108. Aminosäure und der 13. bis 118. Aminosäure von TACI bestehenden Gruppe ausgewählt ist; es sich bei der Linker-Sequenz um 9Gly handelt; und der Immunglobulin-Fc-Anteil von dem Fc der menschlichen oder tierischen Immunglobuline IgG, IgM, IgD und IgA, einschließlich jeweils aller Isotypen, stammt.

3. Fusionsprotein nach Anspruch 1, **dadurch gekennzeichnet, dass** der Immunglobulin-Fc-Anteil von IgG1 stammt.

4. Fusionsprotein nach Anspruch 1, **dadurch gekennzeichnet, dass** die Aminosäuresequenz des Fusionsproteins aus den Sequenzen gemäß SEQ ID NO: 1 bis 3 ausgewählt ist.

5. DNA-Sequenz, codierend das Fusionsprotein nach Anspruch 1.

6. DNA-Sequenz nach Anspruch 5, **dadurch gekennzeichnet, dass** es sich dabei um die Sequenz gemäß SEQ ID NO: 5 bis 7 handelt.

7. Vektor, umfassend die DNA-Sequenz nach Anspruch 5.

8. Zellen, umfassend den Vektor nach Anspruch 7, die für die Expression des entsprechenden Fusionsproteins nach Anspruch 1 verwendet werden und aus eukaryontischen oder prokaryontischen Zellen ausgewählt sind.

9. Verwendung des Fusionsproteins nach Anspruch 1 bei der Herstellung eines Arzneimittels zur Blockierung von BLyS oder APRIL.

10. Pharmazeutische Zusammensetzung, umfassend das Fusionsprotein nach Anspruch 1.

## Revendications

1. Protéine de fusion constituée d'un TACI tronqué et d'un Fc d'immunoglobuline, **caractérisée en ce que** la portion TACI de la protéine de fusion consiste en une séquence de la région amino-terminale de la région extracellulaire partant du 13ème résidu d'acide aminé, en la séquence complète de la région riche en cystéine, et en une séquence partielle de la région tige du TACI ; la portion Fc d'immunoglobuline de la protéine de fusion comprend la région charnière, la région CH2 et la région CH3 ; et la portion TACI et la portion Fc sont fusionnées, directement ou par l'intermédiaire d'une séquence de liaison,
(1) dans laquelle les sites de clivage de PC, sur la position du 9ème, du 12ème, du 120ème et du 135ème acide aminé, ont été éliminés par troncature dans la portion TACI de la protéine de fusion, et
(2) dans laquelle la protéine de fusion a la fonction de blocage de BlyS et d'APRIL.

2. Protéine de fusion selon la revendication 1, **caractérisée en ce que** la portion TACI est choisie dans le groupe consistant en les acides aminés allant du 13ème au 108ème, et les acides aminés allant du 13ème au 118ème de TACI ; la séquence de liaison est 9Gly ; et la portion Fc d'immunoglobuline dérive du Fc des immunoglobulines humaines ou animales IgG, IgM, IgD et IgA, y compris tous les isotypes de chacune d'entre elles.

3. Protéine de fusion selon la revendication 1, **caractérisée en ce que** la portion Fc d'immunoglobuline dérive d'IgG1.

4. Protéine de fusion selon la revendication 1, **caractérisée en ce que** la séquence d'acides aminés de la protéine de fusion est choisie parmi les séquences présentées dans SEQ ID NO:1 à 3.

5. Séquence d'ADN codant pour la protéine de fusion selon la revendication 1.

6. Séquence d'ADN selon la revendication 5, **caractérisée en ce qu'**il s'agit de la séquence présentée dans SEQ ID NO:5 à 7.

7. Vecteur comprenant la séquence d'ADN selon la revendication 5.

8. Cellule comprenant le vecteur selon la revendication 7, qui est utilisée pour l'expression de la protéine de fusion correspondante selon la revendication 1, et qui est choisie parmi les cellules eucaryotes ou les cellules procaryotes.

9. Utilisation de la protéine de fusion selon la revendication 1 pour la fabrication d'un médicament destiné au blocage de BlyS ou d'APRIL.

10. Composition pharmaceutique comprenant la protéine de fusion selon la revendication 1.
